# EUROPEAN PATENT SPECIFICATION

(11) **EP 0 411 124 B1**
(45) Date of publication and mention of the grant of the patent: **13.10.1993**
(21) Application number: 89903231.2
(22) Date of filing: 09.03.1989
(51) Int. Cl.: A61L 27/00

(54) **MEDICAL MATERIAL PERMITTING CELLS TO ENTER THEREINTO AND ARTIFICIAL SKIN**
FÜR ZELLEN EINDRINGBARES MEDIZINISCHES MATERIAL UND KUNSTHAUT
SUBSTANCE MEDICALE DANS LAQUELLE LES CELLULES PEUVENT PENETRER ET PEAU ARTIFICIELLE

(30) Priority: 09.03.1988 JP 53836/88; 25.07.1988 JP 183478/88; 06.09.1988 JP 221337/88
(43) Date of publication of application: 06.02.1991
(73) Proprietor: TERUMO KABUSHIKI KAISHA, Tokyo 151 (JP)
(72) Inventor: YOSHISATO, Katsutoshi, Kanagawa-ken 243-04 (JP); KONISHI, Jun Terumo Kabushiki Kaisha, Shizuoka-ken 417 (JP); KOIDE, Mikio Terumo Kabushiki Kaisha, Shizuoka-ken 417 (JP); OYAMADA, Kaoru Terumo Kabushiki Kaisha, Shizuoka-ken 417 (JP); OHSAKI, Kenichi Terumo Kabushiki Kaisha, Shizuoka-ken 417 (JP); KATAKURA, Takeo Terumo Kabushiki Kaisha, Shizuoka-ken 417 (JP); MORI, Yuichi, Tokyo 151 (JP); TATEBE, Ken Terumo Kabushiki Kaisha 2656-1, Shizuoka-ken 417 (JP)
(74) Representative: Gillard, Marie-Louise
(86) International application number: JP8900258
(87) International publication number: WO8908466

(56) References cited:
- DE-A- 2 631 909
- JP-A-57 168 920
- US-A- 4 522 753

## Description

### [Technical Field]

The present invention relates to cell-penetrable medical material easily assimilable into living tissues and useful as artificial tissues. Further, the present invention also relates to application of this medical material to artificial skin.

### [Background Art]

Implantation is very effective for treating deficiency occurred in part of tissues or irreversible function deficiency. For avoiding the problem of immune incompatibility known as rejection, in this case, it is preferable to transplant the tissue coming from other regions of the patient or his relatives, namely allotransplantation. However, such favorable implanting tissues are not always available. So, such studies for providing implantable artificial tissues have heretofore been made.

First approach for getting artificial tissues free of rejection is to provide the material having low histological reactivity, namely the material which fails to sensitize the tissue and immunocellular system. An example of this approach is the study to enhanse hydrophobicity of the synthetic polymer material represented by polyurethanes.

Second approach is to provide the material which is capable of assimilating rapidly into the tissue before inducing the immunoreaction thereby functioning as a part of an organ. More particularly, it is to construct the tissue similar to connective tissues by penetrating such a cell having the tissue-healing function as fibroblast into the material coming from living bodies such as collagen. Since the new tissue thus formed is no longer not-self, no immune incompatibility would take place. Therefore, it can be said that this approach is more ideal than the first approach.

However, said second approach has the following defects.

Artificial materials consisting of collagen or the like derived from living bodies show high affinity to cellular tissues but would be easily decomposed by collagenase or other enzymes within the living bodies. Therefore, there cannot be sufficiently kept the time for the penetration of fibroblast or the like to construct new tissues. So it is necessary to enforce the physical properties of the material by introducing cross-linking with any means, in order that the material may resist against the decomposition due to collagenase. Dehydrating cross-linking under heating or chemical cross-linking with chemicals can be adopted therefore. Of these cross-linking methods, the dehydrating cross-linking is safer than the chemical treatment, but less resistant against collagenase than the chemical cross-linking. Therefore, it is general that the chemical cross-linking is adopted singly, otherwise a combination of the chemical cross-linking and the dehydrating cross-linking is adopted.

Resistance against collagenase is markedly improved by introducing the cross-linking structure by said methods. For example, when the cross-linking structure is introduced into a collagen by merely dehydrating the collagen under heating at 110°C in vacuum for 24 hours, the cross-linked collagen is dissolved within one day by allowing to stand at 37°C in 3 unit/mℓ collagenase solution. On the contrary, the collagen obtained by introducing the cross-linking structure with an isocyanate type cross-linking agent does not show any change on appearance in 7 days even by allowing to stand still at 37°C in 1000 unit/mℓ collagenase solution.

On the other hand, introduction of said strong cross-linking structure would lower markedly the good affinity to cells or tissues, which is an inherent property of the collagen. Therefore, the penetration of cells would be inhibited to accompany a problem that the new desired tissues could not be formed.

As stated above, it is difficult to suffice both the requirement of good resistance to enzymes and the requirement of good affinity to cells or tissues in materials derived from living bodies such as collagen. Therefore, while the second approach is very attractive, no medical material sufficing the requirements has been developed yet.

The cell-penetrable medical material may be considered to be very useful as coating material for imbedding artificial organs or artificial vessels. However, its use as artificial skin is more realizable and effective.

Artificial skin is an artificial medical material used for coating temporarily or eternally the injured region in order to prevent bacterial infections or overflowing of the humor when any dermal tissue is injured by burning or ambustion. Thus, artificial skin a replacement of autograft skin.

As a wound coating material which can be used for the same purpose as in artificial skin, gauze, absorbent cotton and the like are heretofor used. However, these materials have a disadvantage that they have low inhibitory ability against bacterial infections. In addition, because they absorb rapidly the exudate, the surface of wound would be dried so much and accompanied by pain, bleeding or the like in peeling them. Although sometimes an ointment or the like is used together for avoiding said problem, in this case there would take place another disadvantage that the exudate is so insufficiently absorbed that the surface of wound become excessively moist.

When the surface of wound extends in a broad scope, the following coating films are used. First category includes silicone gauze, silicone rubber film, synthetic fiber sheet such as nylon, teflon or the like having velour-like superficial structure, and other synthetic materials. Second category includes lyophilizated pig's skin, chitin unwoven cloth, collagen film, polyamino acid sponge, mucopolysaccharide complex collagen film, and other materials derived from living bodies.

However, coating films made from said synthetic materials have disadvantages such as poor tight adhesion to the injured region and low steam permeability together with easy inclination to induce cracking. Further, said coating films derived from living bodies show comparatively better adaptability to living bodies but have difficulty in availability of raw material. Moreover, most of them have antigenicity as well as defects such as inclination to deteriorate by bacterial infection or contact with the exudate.

In addition to said coating films, certain complex film made from collagen-treated nylon mesh and silicone film has recently been developed and is commercially available. This complex film has favorable close adhesion to the surface of wound and appropriate water permeability. However, the complex film adheres to the surface of wound, because the granulation tissue enters into the nylon mesh in the course of curing. Since the nylon mesh remains in the granulation tissue without decomposition, after curing the complex film must be peeled together with remarkable pain.

### [Disclosure of the Invention]

A first object of the present invention is to provide cell-penetrable medical material which, because of having the desired resistance to decomposing enzymes, can keep the necessary mechanical strength over a certain period under in vivo circumstances together with favorable affinity to cells and tissues, and also to provide a process for preparing said material.

A second object of the present invention is to provide artificial skin which has a satisfactory function as a replacement of autograft skin, using said medical material, and also to provide a process for preparing it. Thus, artificial skin of the present invention can inhibit bacterial infection and overflowing of the body fluid by coating temporarily or eternally the surface of wound and can accelerate sufficiently repairing the tissue due to cell growth.

The cell-penetrable medical material of the present invention is characterized in consisting essentially of denatured collagen which is obtained by heating, in the presence of water at 50 to 125°C for 20 to 60 minutes, collagen which has been previously subjected to cross-linking treatment.

The process for preparing the cell-penetrable medical material comprises a step of subjecting collagen to cross-linking treatment to give the collagen having cross-linking structure and a step of heating, at 50 to 125°C in the presence of water for 20 to 60 minutes, the collagen which has been previously subjected to cross-linking treatment.

A first artificial skin of the present invention comprises a supporting layer made of fibroin, a wound contacting layer laminated on one side of said supporting layer and a water permeation-controlling layer for controlling water permeation laminated on the other side of said supporting layer, said wound-contacting layer consisting of denatured collagen obtained by heating, in the presence of water at 50 to 125°C for 20 to 60 minutes, the collagen which has been previously subjected to cross-linking treatment. Further, it is preferable to incorporate antibacterial agent into at least one layer of the wound-contacting layer, the supporting layer and the water permeation-controlling layer.

A second artificial skin of the present invention comprises a wound-contacting layer and a water permeation-controlling layer for controlling water permeation laminated on one side of said wound-contacting layer, said wound-contacting layer consisting of denatured collagen obtained by heating, in the presence of water at 50 to 125°C for 20 to 60 minutes, collagen which has been previously subjected to cross-linking treatment. Further, it is preferable to incorporate antibacterial agent into at least one of the wound-contacting layer and the water permeation-controlling layer.

The present invention will be explained in detail as follows :

### Cell-penetrable medical material and a process for preparing it

Cell-penetrable medical material of the present invention is based on the discovery of the fact that cell can easily penetrate into the denatured collagen prepared by heating, in the presence of water, the collagen having cross-linking structure and that the necessary mechanical properties can be kept over a prescribed period under in vivo circumstances.

The collagen having cross-linking structure can be prepared by introducing cross-linking structure in a conventional manner.

As starting collagen to which cross-linking is introduced, there is exemplified the collagen consisting of triple chain helix which is obtained by treating collagen derived from bovine dermis with an acid or alkali. Preferably it is desirous to use the collagen lack of antigenic part (telopeptide) at the terminal of collagen molecule. Such collagen lack of antigenicity and free of this telopeptide is known as Atelocollagen (trademark) in general. Atelocollagen can be prepared by treating the starting collagen with an acid or alkali, as stated above, and then with pepsin specifically working on telopeptide. More preferably, these starting collagens are used as fibrous collagen. Such fibrous collagens can be prepared by neutralizing the collagen consisting of triple chain helix with phosphate buffer at 37°C. Thereby the water soluble and dispersed triple chain helix structure is reconstructed into a fibrous structure having periodicity, whereby the collagen is insolubilized.

For introducing cross-linking structure into the collagen, there are adopted a method of dehydrating the collagen and a method of treating the collagen with a cross-linking agent. The dehydrating is performed by heating the collagen at 50 to 180°C under vacuum of not exceeding 0.05 Torr. for 1 to 24 hours, preferably 100 to 120°C under said vacuum for 2 to 8 hours. When treated with a cross-linking agent, the cross-linking agent used is not particularly limited. For example, there are exemplified aldehyde type cross-linking agents such as glutaraldehyde, isocyanate type cross-linking agent such as hexamethylene diisocyanate and carbodiimide type cross-linking agent such as 1-ethyl-3 (3-dimethylaminopropyl)-carbodiimide hydrochloride.

Introduction of such cross-linking structure improves mechanical strength of the collagen and raises resistance against enzymatic decomposition. This effect is more remarkable in fibrous collagen than in dispersed type triple chain collagen. Thus, introduction of such cross-linking structure into fibrous collagen leads to marvelous improvement of physical properties such as mechanical strength by synergistic action between fibrous structure and cross-linking structure. When the degree of cross-linking to be introduced into the collagen is too low, no sufficient physical strength can be obtained. When it is too high on the contrary, the structure and favorable properties of collagen are damaged. Therefore, it is necessary to set appropriately the cross-linking degree depending upon various conditions. For example, when any cross-linking agent is used for fibrous collagen, appropriate cross-linking degree can be obtained by using in general 0.01 to 5% (w/v), preferably 1 to 3% (w/v) of cross-linking agent.

Subsequently, the collagen containing said cross-linking structure thus obtained is denatured under heating. Heating is made in the presence of water at 50 to 125°C, preferably 90 to 121°C over a period of 20 minutes to 1 hour. Since the helix structure of the raw collagen is modified by this heating into random coil, the helix content rate becomes an index of denaturation degree. More accurately, the denaturation degree is represented by [1-(Helix content rate)]. Appropriate helix content rate of denatured collagen used in the present invention is 0 to 80%, preferably 40 to 70%.

Further, said helix content rate can be measured with circular dichroism spectrometer (CD) or infra red spectrophotometer [P. L. Gorden et at., Macromoles, 1 (6), 954, 1974; Masanobu Nakura, Hashimoto, et al., Polymer Article Collection, 41 (8), 473, 1984]. Values of the helix content rate used in the present application were calculated on the basis of data of this spectrometer. Moreover, it has been found from the result of electrophoresis that a part of the collagen molecule is cleaved in said denatured collagen.

As described above, it seems that cells can penetrate easily into the denatured collagen, because the helix is converted into random coil and a part thereof is cleaved on the way in the denatured collagen molecule used in the present invention. On the contrary, it is apt to undergo decomposition with collagenase through the denaturation and is decomposed comparatively rapidly. However, as described above, cross-linking structure is introduced prior to said denaturation, and resistance to enzymatic decomposition with collagenase or the like is afforded. By contribution of this cross-linking structure, there can be kept the time for penetration of fibroblast or the like and for construction of new tissues. Thus, the part having the cross-linking structure forms a skeleton structure resistant to decomposing action of enzymes, in the collagen after the thermal denaturation.

As stated above, the medical material of the present invention can attain excellent cell-penetration, and on the other hand sufficient strength can be kept until dermis-like connective tissues can be formed by penetration of cells. Further, since the whole is finally decomposed and absorbed by undergoing enzymatic action, it can be completely assimilated in vivo.

The medical material of the present invention can be made in an appropriate form either before or after introducing the cross-linking structure into the collagen. In any case, thermal denaturation in the presence of water can be made under the appropriate form.

The following methods can be adopted for making the appropriate form. If the solution of collagen before cross-linking treatment is used, there is used a method of forming films by solvent-casting or a method of forming porous sponge by lyophilization. When aqueous collagen solution after cross-linking treatment is used, a method can be adopted, in which the collagen solution is gelled and the gel thus obtained is then shaped.

### Artificial Skin

Artificial skin of the present invention is a multi-layer complex film comprising said cell-penetrable medical material.

### 〈First Artificial Skin〉

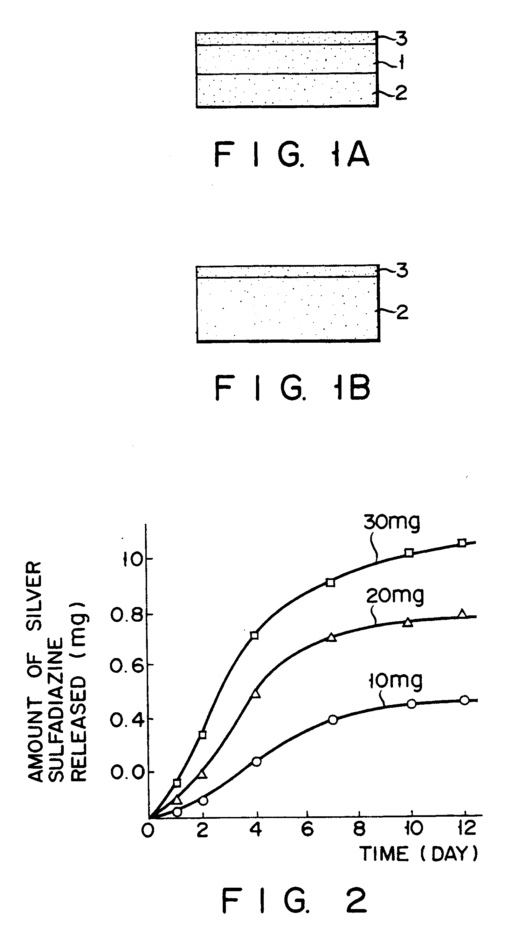

This artificial skin is typically a three-layered complex film as shown in Fig. 1A. In Fig. 1, 1 is a supporting layer made from fibroin film. Below said supporting layer 1 a wound-contacting layer 2 is laminated, and over the supporting layer 1 a water permeation controlling layer 3 is laminated. Said cell-penetrable medical material is used in wound-contacting layer 2. These layers are hereafter explained in detail, respectively.

### (1) Wound-contacting Layer 2

For wound-contacting layer 2 is used said cell-penetrable medical material. Thus, wound-contacting layer 2 is made of denatured collagen obtained by heat-treating the collagen having cross-linking structure. Composition and the like of this wound-contacting layer are not explained in detail, because they are described above. Necessarily, this wound-contacting layer can take various forms such as film, sponge or the like, as described above on those of medical materials.

Wound-contacting layer 2 has a function that it softly protects the surface of the wound by covering directly the wound, lowers pain, affords appropriate water and inhibits bacterial infection. Further, it accelerates new formation of the tissues with cell growth and stimulates curing. Said wound-contacting layer 2 made of cell-penetrable materials has all these functions and in particular exceeds markedly in accelerating new formation of the tissues. Thus, once it is applied on the surface of the wound, inflammatory cells such as macrophage, neutrophile or the like are infiltrated together with rapid invasion of fibroblast. As a result, dermis-like connective tissues are constructed to accelerate curing of the wound. Since this invasion of cells are described in detail on the medical material above, its explanation is abridged. Further, this wound-contacting layer 2 is finally decomposed by enzymes to be absorbed into the living body. Accordingly, it is not necessary to accompany marked pain for removing the artificial skin, as seen in known artificial skins.

### (2) Supporting Layer 1

Supporting layer 1 enforces mechanical strength of wound-contacting layer 2 and makes the penetration of cells into wound-contacting layer 2 smooth.

As stated above, the wound-contacting layer has prescribed resistance against collagenase by using fibrous collagen together. However, since the mechanical strength as coating materials are still insufficient and finally the would-contacting layer is assimilated into the living body, any supporting body for protecting it from the outside stimulations is necessary. So, supporting layer 1 is needed to have mechanical strength exceeding certain level. At the same time, supporting layer 1 should not inhibit invasion of cells into would-contacting layer 2. Fibroin is used as material for sufficing these requirements.

Fibroin is material derived from living bodies and is a protein which is a main constituent of silk. As appreciated from the fact that silk is used for suture for surgical operation, fibroin is a protein excellent in stability in living bodies. Aqueous solution of fibroin can be prepared by dissolving fibroin in a concentrated neutral solution of salts such as lithium bromide or calcium chloride and subjecting the solution to dialysis or the like. This aqueous solution of fibroin is freezed at -18°C to 0°C and defrosted to form β-type crystalline structure, thereby giving water-insoluble porous unwoven cloth [Jun Umagoshi, Kobunshi Kagaku (Polymer Chemistry), 30, 582 (1973)]. This porous unwoven cloth of fibroin shows more excellent stability in living bodies than ordinary collagen having cross-linking structure. So, it is appropriately used as a supporting layer of artificial skin used for coating the surface of would over a long period of time.

### (3) Water Permeation Controlling Layer 3

Water permeation controlling layer 3 functions to control water content on the surface of wound during artificial skin is applied on the surface of wound. Pooling exudate on the surface of wound can be avoided while keeping the surface of wound wet and moist, by ensuring appropriate steam permeation with this water permeation controlling layer 3. At the same time, transudation of the protein ingredients in the exudate outside can be prevented, thereby making favorable circumstances for repairing tissues. It is heretofore well known that such water permeation controlling layer is used in wound coating material, and these known materials can be also used for artificial skin of the present invention. Thus, a film of non-toxic material having about 0.1 to 1 mg/cm²/hour of water flux can be used as such water permeation controlling layer 3. Its appropriate thickness is about 5 to 200 µm. Non-toxic materials include silicone resin, polyacrylate ester, polymethacrylate ester, polyurethane and the like. In particular, silicone resin is preferred.

Said artificial skin having three layers can exhibit very excellent therapeutic effect as artificial skin, because wound-contacting layer 2 has favorable cell-penetration and appropriate resistance against enzymic decomposition, satisfactory mechanical strength is given by supporting layer 1 and appropriate water content is kept on the surface of wound by water permeation controlling layer 3. Since an improvement of cell-penetration and resistance to enzymes, both of which were heretofore incompatible, have been attained in wound-contacting layer 2, there can be obtained by far more excellent therapy accelerating effect than known wound coating materials.

In preferred embodiments of said artificial skin, antibacterial agent is incorporated in at least one of such wound-contacting layer 2, supporting layer 1 and water permeation controlling layer 3. Appropriate antibacterial agents include silver sulfadiazine, gentamycin and silver nitrate. However, various other antibacterial agents can be used without limiting to them. Incorporation of such antibacterial agents is a means for preventing effectively bacterial infection.

Bacterial infection is apt to occur in broad ambustion or serious ambustion (for example, degree III ambustion). Cream base containing an antibacterial agent has heretofore been used for preventing such bacterial infection. However, when cream base is used together with known wound coating material such as gauze or bandage, about 57% of the applied cream soaks into gauze or the like together with the exudate and about 21% of the cream only gets to the surface of wound. Further, the cream has to be applied everyday to need troublesome labour. On the contrary, if the artificial skin of the present invention contains an antibacterial agent, it can release gradually the antibacterial agent in a certain period.

Therefore, the antibacterial agent can act continuously without exposing the surface of wound to ambient air and without troublesome labour in applying the antibacterial agent to the wound everyday, whereby bacterial infection can be prevented effectively in combination with bacterial penetration-preventive effect of the water permeation controlling layer.

The following experiment was performed for examining the aspect of releasing the antibacterial agent contained in water permeation controlling layer 3. At first, three silicone films (about 0.15 g) of 20 µm thick in size (5 cm × 5 cm) were prepared, which respectively contain 10 mg, 20 mg and 30 mg of silver sulfadiazine (AgSD). These respective samples were dipped in 100 ml of distilled water, and the amount of AgSD eluted was measured in the lapse of time, respectively. The result is shown in Fig. 2. In this Figure, the abscissa shows the lapse of time (day) and the ordinate shows the cumulative amount of AgSD eluted. It is observed from the result that AgSD contained in the silicone film is gradually released to attain the gradual releasability enough to get the effect described above.

When a main object is to prevent bacterial invasion from outside and it is necessary to add antibacterial agent into any one of these layers, it is preferred to select water permeation controlling layer 3. On the other hand, when the surface of wound is already infected by bacteria and a lot of antibacterial agent is needed to be applied on the wound, it is desirable to incorporate antibacterial agent into either wound-contacting layer 2 or supporting layer 1. Of course, one can incorporate antibacterial agent into any two or three members of wound-contacting layer 2, supporting layer 1 and water permeation controlling layer 3.

### 〈Second Artificial Skin〉

This artificial skin is a complex film consisting of wound-contacting layer 2 and water permeation controlling layer 3 as typically shown in Fig. 1B. Thus, the second artificial skin is lack of supporting layer 1, but otherwise quite same as described in first artificial skin.

### Production of Artificial Skin

Said first artificial skin can be prepared easily, for example, according to the following steps a) to h).
a) At first aqueous solution of collagen (preferably fibrous collagen) is prepared.
b) Alternatively, porous fibroin film to use as supporting layer 2 is prepared according to the method already described.
c) Subsequently, said collagen solution is contained in a certain vessel, and said fibroin film is set on the liquid surface and lyophilized. Thereby a laminate consisting of supporting layer 1 made of fibroin film and porous collagen layer 2' is obtained.
d) Cross-linking structure is introduced into porous collagen layer 2' obtained in Step C). The method for the cross-linking procedure may be either dehydration by heating or treatment with cross-linking agent.
e) A solution to afford water-permeable film such as silicone or the like is developed on a teflon substrate or the like having peeling surface, thereby giving viscous thin film.
f) Said laminate subjected to cross-linking treatment in Step d) is set on the viscous thin film of Step e). In this case supporting layer 2 is set so as to contact with the viscous thin layer.
g) Said viscous thin film is dried up to hardening and heated at 50 to 180°C under vacuum not exceeding 0.05 Torr for 1 to 24 hours. Thereby said viscous thin film is dried and water permeation controlling layer 3 is formed. At the same time said controlling layer 3 is bound with said supporting layer 1 as a whole.
h) three layers-laminate obtained in Step g) is heated at 50 to 125°C, preferably 90 to 121°C for 20 minutes to 1 hour in the presence of water. Thereby the collagen containing cross-linking structure which constitutes porous layer 2' is denatured and converted into the desirous wound-contacting layer 2. Thus artificial skin consisting of three layers structure (Fig. 1A) is obtained.

Further, cross-linking treatment may be effected under solution in Step a) in place of the cross-linking treatment in Step d).

Moreover, the porous collagen film (preferably fibrous collagen having cross-linking structure) may be used in place of fibroin film as supporting layer 1 above. The porous film of fibrous collagen having cross-linking structure is prepared by lyophilizing aqueous solution of fibrous cross-linking collagen obtained above. However, since the cross-linking collagen film used in supporting layer 1 is also denatured by Step g) in this case, supporting layer 1 is not distinctive from wound-contacting layer 2. So, artificial skin obtained in this case is not three layers-structure (Fig. 1 A) but two layers-structure (Fig. 1 B).

Artificial skin in two layers (Fig. 1 B) can be easily prepared also by the following Steps a) to f).
a) At first aqueous solution of collagen (preferably fibrous collagen) is prepared. Then, cross-linking agent is added to said aqueous solution to give aqueous solution of collagen having cross-linking structure.
b) Aqueous solution of cross-linked collagen thus obtained is gelled and formed into the shape of wound-contacting layer 2'.
c) Solution of such a substance to afford water permeable film as silicone or the like is developed on a teflon substrate or the like having peeling surface to give viscous thin film.
d) Cross-linked collagen in the shaped form obtained in Step b) is set on this viscous thin film.
e) Said viscous thin film is dried up to hardening and heated at 50 to 180°C under vacuum not exceeding 0.05 Torr, for 1 to 24 hours. Thereby said viscous thin film is dried to give water permeation-controlling layer 3 and at the same time said water permeation-controlling layer 3 is bound to said wound-contacting layer 2' as a whole.
f) Said two layers-laminate obtained in Step e) is heated at 50 to 125°C, preferably 90 to 121°C for 20 minutes to 1 hour in the presence of water. Thereby the collagen having cross-linking structure which constitutes gelled layer 2' is denatured and converted into the desired wound-contacting layer 2. Thus, there is obtained artificial skin having two layers structure in Fig. 1B.

Further, denaturation of the cross-linked collagen may be performed under the shaped form obtained in Step b) in place of the denaturation in Step f).

Furthermore, even in any preparing processes above, favorable artificial skin containing said antibacterial agent can be prepared by incorporating antibacterial agent into at least one of the collagen solution to form wound-contacting layer 2, said fibroin film or cross-linked fibrous collagen film to be used as a supporting layer, or said solution containing said substance to afford water permeable film.

### [Brief Explanation of the Drawing]

Fig. 1A and Fig. 1B are sectional views showing multi-layer structures of the artificial skin in the present invention; and
Fig. 2 is a graph showing the result of tests in which releasing aspect of the antibacterial agent incorporated into the water permeation-controlling layer of the artificial skin in Fig. 1 is examined.

### [Best Mode of the Invention]

Now preferred and practical embodiments of the present invention are illustratively shown in the following examples.

### 〈A〉 Example of Cell-penetrable Medical Material and Production thereof (Example 1 to Example 2)

### Example 1

One gram of Atelocollagen was dissolved in dilute hydrochloric acid (pH 3.0) to give 0.3% (w/v) solution. This solution was mixed with phosphate buffer with stirring in a thermostatt of 4°C to give collagen solution containing 0.1% (w/v) of Atelocollagen, 30 mM disodium phosphate and 100 mM sodium chloride. This solution was left in a thermostatt of 37°C for 1 day to give fibrous Atelocollagen solution. this solution was concentrated by centrifuging at 5000 r.p.m. for 10 minutes to give 0.3% (w/v) solution of fibrous Atelocollagen. This solution was freezed rapidly at -30°C and lyophilized to give a sponge. This sponge was dipped in 1% (w/v) solution of hexamethylene diisocyanate in ethanol at room temperature for 24 hours to introduce cross-linking structure. After non-reacted isocyanate was removed by washing with water, the sponge was dipped in distilled water in sterilizing bottle and heated at 121°C for 30 minutes. Further, it was lyophilized and heated at 110°C for 2 hours in vacuum for sterlization to give medical material.

### Example 2

Preparation was performed in the same manner as in Example 1. Sponge containing cross-linking structure was dipped in distilled water and heated at 90°C for 30 minutes. After lyophilizing, the sponge was heated at 110°C under vacuum for 2 hours for sterilization to give medical material.

### Control Example 1

Preparation was performed in the same manner as in Example 1. Sponge containing cross-linking structure was dipped in distilled water and heated at 40°C for 30 minutes. After lyophilizing, the sponge was heated t 110°C under vacuum for 2 hours to give medical material.

### Control Example 2

Preparation was performed in the same manner as in Example 1. Sponge containing cross-linking structure was heated at 110°C under vacuum for 2 hours for sterilizing to give medical material.

### Experiment 1

### Test of imbedding subcutaneously various medical materials:

Tests of imbedding subcutaneously in rat for observing tissue adaptability on sponges obtained in Example 1-2 and Control Example 1-2 were performed. Sponges in size of 1 cm × 1 cm × 1.5 mm obtained in Example 1-2 and control Example 1-2 were inserted into the opening in about 1.5 cm made by cutting deep on the back of rats, and the wound was closed by stitching with a suture thread. Seventh and 14th days the rats were killed, and dorsal skin tissues were cut off. Cell-penetration into the sponges was observed according to ordinary method of making tissue slices. Table 1 shows the results. As clearly shown in Table 1. Cell-penetration was found to be markedly improved by heating in the presence of water.

**Table 1**

| Cell-penetration into Sponge | | |
|---|---|---|
| Sample | Observation for 7 days | Observation for 14 days |
| Example 1 | Penetration of neutrophil and monocyte into inside of sponges observed | Granulation and cellular infiltration observed |
| Example 2 | Ditto | Ditto |
| Control Example 1 | Cell-penetration hardly observed/slight encapsulation present | No penetration into inside of sponges observed/Surroundings of sponges were encapsulated by strong connective tissues |
| Control Example 2 | Ditto | Ditto |

### Experiment 2

### Decomposition of medical material by enzymes:

Each sponges obtained in Example 1-2 and Control example 1-2 was incubated at 37°C for 24 hours in 100 unit/ml solution of collagenase derived from bacteria. Amount of hydroxyproline of the collagen eluted into the solution was measured in the lapse of time. Decomposing ratio (%) was calculated by comparing the amount of eluted hydroxyproline with the initial amount of hydroxyproline of the collagen. Table 2 shows the result.

**Table 2**

| Decomposition of Sponges by Enzymes | | |
|---|---|---|
| Sample | Eluted amount of the collagen (%) | |
| | 3rd day | 7th day |
| Example 1 | 2 | 7 |
| Example 2 | 1 | 5 |
| Control Example 1 | 0 | 3 |
| Control Example 2 | 0 | 4 |

It is evident from Table 2 that the medical material of the present invention has almost the same resistance against enzymes as in Control Example.

### 〈B〉 Examples of Artificial Skin and production thereof (Example 3-5)

### Example 3

### preparation of Fibroin Matrix:

Purified silk was dissolved in 8 M aqueous solution of lithium bromide, and the solution was put in a cellophane tube and dialyzed to water. After confirming that lithium bromide was completely removed, the resultant aqueous solution of fibroin was flowed into a polystyrene vessel, freezed at -10°C for 24 hours and defreezed at room temperature. After confirming that it became unwoven cloth, it was lyophilized.

### Example 4

### Preparation of Artificial Skin:

1.0% (w/v) solution of fibrous Atelocollagen was prepared in the same manner as in Example 1. This solution was poured into a stainless vat. Further a sponge of fibroin matrix obtained in Example 3 above was gradually put thereon, and the sponge floated on the upper part of the solution. It was rapidly freezer at -30°C under these conditions, sufficiently freezed and lyophilized at -40°C under vacuum not exceeding 0.1 Torr. The sponge was dipped in 1% (w/v) solution of hexamethylene diisocyanate in ethanol at room temperature for 24 hours to introduce cross-linking structure. After the non-reacted isocyanate was washed out with water, the sponge was dipped in distilled water in a sterilized bottle, heated at 121°C for 20 minutes and lyophilized, thereby resulting the sponge in two layers-structure of fibroin matrix and denatured fibrous Atelocollagen matrix. Then 50% solution of silicone adhesive (Silastic type A; Dow Corning) in hexane was applied on a teflon plate with an applicator for making film. Just after application, said sponge was put thereon so that the fibroin matrix would contact with the silicone side, allowed to stand at room temperature for 10 minutes and heated at 60°C in an oven for at least 1 hour for hardening. Further it was heated at 110°C under vacuum not exceeding 0.05 Torr, for 2 hours for sterilization to give the desired artificial skin.

### Example 5

### Preparation of Artificial Skin containing Antibacterial Agent:

Preparation was performed in the same manner as in Example 4 except the following. Thus 50% solution of silicone adhesive (Silastic type A: Dow Corning) in hexane containing 50 mg of silver sulfadiazine (AgSD) was applied on a teflon plate with an applicator for making film. The desired artificial skin was prepared by effecting the same procedure as in Example 4 above.

## Claims

1. Cell-penetrable medical material characterized in being made of denatured collagen prepared by heating, in the presence of water at 50 to 125°C for 20 to 60 minutes, collagen which has been previously subjected to cross-linking treatment.

2. Medical material according to claim 1, in which said denatured collagen is devoid of the moiety having antigenicity at the terminal of collagen molecule.

3. Medical material according to claim 1, in which said denatured collagen contains 0 to 80% of helix.

4. Cell-penetrable medical material characterized in being made from denatured collagen prepared by heating, in the presence of water at 50 to 125°C for 20 to 60 minutes, fibrous collagen which has been previously subjected to cross-linking treatment.

5. Medical material according to claim 4, in which said denatured collagen is devoid of the moiety having antigenicity at the terminal of collagen molecule.

6. Medical material according to claim 4, in which said denatured collagen contains 0 to 80% of helix.

7. Medical material according to claim 4, in which it is in the form of film or sponge.

8. Process for preparing cell-penetrable medical material comprising :
a) a step of subjecting collagen to cross-linking treatment to give the collagen having cross-linking structure, and
b) a step of heating, in the presence of water, the collagen having a cross-linking structure at 50 to 120°C for 20 to 60 minutes.

9. Process according to claim 8, in which fibrous collagen is used as the collagen to be subjected to cross-linking treatment in Step a).

10. Process according to claim 8, in which collagen devoid of the moiety having antigenicity at the terminal of collagen molecule is used as the collagen to be cross-linked in Step a).

11. Process according to claim 8, in which the cross-linking treatment in Step a) is dehydrating procedure under heating.

12. Process according to claim 8, in which the cross-linking treatment in Step a) is treatment with a cross-linking agent.

13. Process according to claim 12, in which concentration of said cross-linking agent is 0.01 to 5% (w/v).

14. Artificial skin comprising :
a supporting layer made from fibroin ;
a wound-contacting layer laminated on one side of said supporting layer and
a water permeation-controlling layer laminated on the other side of said supporting layer for controlling water permeation
said wound-contacting layer being made of denatured collagen prepared by heating, in the presence of water at 50 to 125°C for 20 to 60 minutes, collagen which has been previously subjected to cross-linking treatment.

15. Artificial skin according to claim 14, in which said denatured collagen is devoid of the moiety having antigenicity at the terminal of collagen molecule.

16. Artificial skin according to claim 14, in which said denatured collagen is prepared by heating, in the presence of water at 50 to 125°C for 20 to 60 minutes, fibrous collagen which has been previously subjected to cross-linking treatment.

17. Artificial skin according to claim 14, in which said wound-contacting layer takes sponge form.

18. Artificial skin according to claim 14, in which said denatured collagen contains 0 to 80% of helix.

19. Artificial skin according to claim 14, in which fibroin of said supporting layer takes water-insoluble unwoven porous form.

20. Artificial skin according to claim 14, in which said water permeation-controlling layer is made of non-toxic materials selected from the group consisting of silicone resin, polyacrylate ester, polymethacrylate ester and polyurethane.

21. Artificial skin according to claim 14, in which said water permeation-controlling layer has water flux of about 0.1 to 1mg/cm²/hour.

22. Artificial skin according to claim 14, in which at least one of said wound-contacting layer, supporting layer and water permeation controlling layer contains an antibacterial agent.

23. Artificial skin according to claim 22, in which said antibacterial agent is selected from the group consisting of silver sulfadiazine, gentamycin and silver nitrate.

24. Artificial skin comprising :
a wound-contacting layer ; and
a water permeation-controlling layer laminated on one side of said wound-contacting layer for controlling water permeation,
said wound-contacting layer being made of denatured collagen obtained by heating, in the presence of water at 50 to 125°C for 20 to 60 minutes, collagen which has been previously subjected to cross-linking treatment.

25. Artificial skin according to claim 24, in which said denatured collagen is devoid of the moiety having antigenicity at the terminal of collagen molecule.

26. Artificial skin according to claim 24, in which said denatured collagen is prepared by heating, in the presence of water at 50 to 125°C for 20 to 60 minutes, fibrous collagen which has been previously subjected to cross-linking treatment.

27. Artificial skin according to claim 24, in which said wound-contacting layer takes sponge form.

28. Artificial skin according to claim 24, in which said denatured collagen contains 0 to 80% of helix.

29. Artificial skin according to claim 24, in which said water permeation-controlling layer is made of non-toxic material selected from the group consisting of silicone resin, polyacrylate ester, polymethacrylate ester and polyurethane.

30. Artificial skin according to claim 24, in which said water permeation-controlling layer has water flux of about 0.1 to 1 mg/cm²/hour.

31. Artificial skin according to claim 24, in which at least one of wound-contacting layer and water permeation-controlling layer contains an antibacterial agent.

32. Artificial skin according to claim 31, in which said antibacterial agent is selected from the group consisting of silver sufadiazine, gentamycin and silver nitrate.

## Patentansprüche

1. Für Zellen durchdringliches medizinisches Material, dadurch gekennzeichnet, daß es aus denaturiertem Kollagen, das durch 20- bis 60-minütiges Erwärmen von zuvor einer Vernetzungsbehandlung unterworfenem Kollagen in Gegenwart von Wasser auf 50 - 125°C hergestellt wurde, gebildet ist.

2. Medizinisches Material nach Anspruch 1, wobei dem denaturierten Kollagen die Antigenizität aufweisende Einheit am Ende des Kollagenmoleküls fehlt.

3. Medizinisches Material nach Anspruch 1, wobei das denaturierte Kollagen 0 - 80% Helix enthält.

4. Für Zellen durchdringliches medizinisches Material, dadurch gekennzeichnet, daß es aus denaturiertem Kollagen, das durch 20- bis 60-minütiges Erwärmen von zuvor einer Vernetzungsbehandlung unterworfenem faserförmigem Kollagen in Gegenwart von Wasser auf 50 - 125°C hergestellt wurde, gebildet ist.

5. Medizinisches Material nach Anspruch 4, wobei dem denaturierten Kollagen die Antigenizität aufweisende Einheit am Ende des Kollagenmoleküls fehlt.

6. Medizinisches Material nach Anspruch 4, wobei das denaturierte Kollagen 0 - 80% Helix enthält.

7. Medizinisches Material nach Anspruch 4, wobei dieses in Film- oder Schwammform vorliegt.

8. Verfahren zur Herstellung eines von Zellen durchdringlichen medizinischen Materials durch
a) eine Stufe, in der Kollagen einer Vernetzungsbehandlung unterworfen wird, um dem Kollagen eine Vernetzungsstruktur zu verleihen, und
b) eine Stufe, in der das Kollagen mit Vernetzungsstruktur in Gegenwart von Wasser 20 - 60 min lang auf 50 - 120°C erwärmt wird.

9. Verfahren nach Anspruch 8, wobei in Stufe a) als einer Vernetzungsbehandlung zu unterwerfendes Kollagen ein faserförmiges Kollagen verwendet wird.

10. Verfahren nach Anspruch 8, wobei in Stufe a) als das zu vernetzende Kollagen ein Kollagen verwendet wird, dem die Einheit mit Antigenizität am Ende des Kollagenmoleküls fehlt.

11. Verfahren nach Anspruch 8, wobei es sich bei der Vernetzungsbehandlung in Stufe a) um eine Dehydratisierungsmaßnahme unter Erwärmen handelt.

12. Verfahren nach Anspruch 8, wobei es sich bei der Vernetzungsbehandlung in Stufe a) um eine Behandlung mit einem Vernetzungsmittel handelt.

13. Verfahren nach Anspruch 12, wobei die Konzentration an dem Vernetzungsmittel 0,01 bis 5% (g/v) beträgt.

14. Künstliche Haut, umfassend
eine Trägerschicht aus Fibroin;
eine auf einer Seite der Trägerschicht auflaminierte Wundkontaktschicht und
eine auf die andere Seite der Trägerschicht zur Steuerung des Wasserdurchtritts laminierte, den Wasserdurchtritt steuernde Schicht,
wobei die Wundkontaktschicht aus einem denaturierten Kollagen gebildet ist, das durch 20- bis 60-minütiges Erwärmen von zuvor einer Vernetzungsbehandlung unterworfenem Kollagen in Gegenwart von Wasser auf 50 - 125°C hergestellt wurde.

15. Künstliche Haut nach Anspruch 14, wobei dem denaturierten Kollagen die Antigenizität aufweisende Einheit am Ende des Kollagenmoleküls fehlt.

16. Künstliche Haut nach Anspruch 14, wobei das denaturierte Kollagen durch 20- bis 60-minütiges Erwärmen von zuvor einer Vernetzungsbehandlung unterworfenem faserförmigem Kollagen in Gegenwart von Wasser auf 50 - 125°C hergestellt wurde.

17. Künstliche Haut nach Anspruch 14, wobei die Wundkontaktschicht schwammförmig ist.

18. Künstliche Haut nach Anspruch 14, wobei das denaturierte Kollagen 0 - 80% Helix enthält.

19. Künstliche Haut nach Anspruch 14, wobei das Fibroin der Trägerschicht in in Wasser unlöslicher poröser Vliesform vorliegt.

20. Künstliche Haut nach Anspruch 14, wobei die den Wasserdurchtritt steuernde Schicht aus nicht-toxischen Materialien, ausgewählt aus Silikonharz, Polyacrylatester, Polymethacrylatester und Polyurethan, gebildet ist.

21. Künstliche Haut nach Anspruch 14, wobei die den Wasserdurchtritt steuernde Schicht einen Wasser(zu)fluß von etwa 0,1 - 1 mg/cm²/h aufweist.

22. Künstliche Haut nach Anspruch 14, wobei mindestens eine der Wundkontaktschicht, Trägerschicht und den Wasserdurchtritt steuernde Schicht ein antibakterielles Mittel enthält.

23. Künstliche Haut nach Anspruch 22, wobei das antibakterielle Mittel aus der Gruppe Silbersulfadiazin, Gentamycin und Silbernitrat ausgewählt ist.

24. Künstliche Haut, umfassend
eine Wundkontaktschicht und
eine den Wasserdurchtritt steuernde Schicht, die zur Steuerung des Wasserdurchtritts auf eine Seite der Wundkontaktschicht laminiert ist,
wobei die Wundkontaktschicht aus denaturiertem Kollagen, das durch 20- bis 60-minütiges Erwärmen eines zuvor einer Vernetzungsbehandlung unterworfenen Kollagens in Gegenwart von Wasser auf 50 - 125°C hergestellt wurde, gebildet ist.

25. Künstliche Haut nach Anspruch 24, wobei dem denaturierten Kollagen die Antigenizität aufweisende Einheit am Ende des Kollagenmoleküls fehlt.

26. Künstliche Haut nach Anspruch 24, wobei das denaturierte Kollagen durch 20- bis 60-minütiges Erwärmen von zuvor einer Vernetzungsbehandlung unterworfenem faserförmigem Kollagen in Gegenwart von Wasser auf 50 - 125°C hergestellt wurde.

27. Künstliche Haut nach Anspruch 24, wobei die Wundkontaktschicht schwammförmig ist.

28. Künstliche Haut nach Anspruch 24, wobei das denaturierte Kollagen 0 - 80% Helix enthält.

29. Künstliche Haut nach Anspruch 24, wobei die den Wasserdurchtritt steuernde Schicht aus nicht-toxischen Materialien, ausgewählt aus Silikonharz, Polyacrylatester, Polymethacrylatester und Polyurethan, gebildet ist.

30. Künstliche Haut nach Anspruch 24, wobei die den Wasserdurchtritt steuernde Schicht einen Wasser(zu)fluß von etwa 0,1 - 1 mg/cm²/h aufweist.

31. Künstliche Haut nach Anspruch 24, wobei mindestens eine der Wundkontaktschicht und den Wasserdurchtritt steuernde Schicht ein antibakterielles Mittel enthält.

32. Künstliche Haut nach Anspruch 31, wobei das antibakterielle Mittel aus der Gruppe Silbersulfadiazin, Gentamycin und Silbernitrat ausgewählt ist.

## Revendications

1. Matière médicale susceptible de pénétation par des cellules, caractérisée en ce qu'elle est en collagène dénaturé préparé par chauffage, en présence d'eau, entre 50 et 125°C, pendant 20 à 60 min, de collagène qui a été préalablement soumis à un traitement réticulant.

2. Matière médicale selon la revendication 1, dans laquelle ledit collagène dénaturé est dépourvu de la fraction ayant un pouvoir antigénique à l'extrémité terminale de la molécule de collagène.

3. Matière médicale selon la revendication 1, dans laquelle ledit collagène dénaturé contient 0 à 80 % d'hélice.

4. Matière médicale susceptible de pénétration par des cellules, caractérisée en ce qu'elle est en collagène dénaturé préparé par chauffage, en présence d'eau, entre 50 et 125°C, pendant 20 à 60 min, de collagène fibreux qui a été préalablement soumis à un traitement réticulant.

5. Matière médicale selon la revendication 4, dans laquelle ledit collagène dénaturé est dépourvu de la fraction possédant un pouvoir antigénique à l'extrémité terminale de la molécule de collagène.

6. Matière médicale selon la revendication 4, dans laquelle ledit collagène dénaturé contient 0 à 80 % d'hélice.

7. Matière médicale selon la revendication 4, laquelle se présente sous forme de film ou de matière spongieuse.

8. Procédé de préparation d'une matière médicale susceptible de pénétration par des cellules, comprenant :
a) une étape consistant à soumettre du collagène à un traitement réticulant pour obtenir le collagène ayant une structure réticulante et
b) une étape consistant à chauffer le collagène ayant une structure réticulante en présence d'eau, entre 50 et 120°C pendant 20 à 60 min.

9. Procédé selon la revendication 8, dans lequel du collagène fibreux est utilisé en tant que collagène à soumettre au traitement réticulant de l'étape a).

10. Procédé selon la revendication 8, dans lequel du collagène dépourvu de la fraction ayant un pouvoir antigénique à l'extrémité terminale de la molécule de collagène est utilisé comme collagène à réticuler au cours de l'étape a).

11. Procédé selon la revendication 8, dans lequel le traitement réticulant de l'étape a) est une technique de déshydratation sous chauffage.

12. Procédé selon la revendication 8, dans lequel le traitement réticulant de l'étape a) est un traitement par un agent réticulant.

13. Procédé selon la revendication 12, dans lequel la concentration dudit agent réticulant est comprise entre 0,01 et 5 % (p/v).

14. Peau artificielle comprenant :
une couche support en fibroïne ;
une couche en contact avec la plaie, appliquée sur une face de ladite couche support et
une couche contrôlant la perméation de l'eau, appliquée sur l'autre face de ladite couche support en vue du contrôle de la perméation de l'eau,
ladite couche en contact avec la plaie étant en collagène dénaturé, préparé par chauffage, en présence d'eau, entre 50 et 125°C, pendant 20 à 60 min, de collagène qui a été préalablement soumis à un traitement réticulant.

15. Peau artificielle selon la revendication 14, dans laquelle ledit collagène dénaturé est dépourvu de la fraction possédant un pouvoir antigénique à l'extrémité terminale de la molécule de collagène.

16. Peau artificielle selon la revendication 14, dans laquelle ledit collagène dénaturé est préparé par chauffage, en présence d'eau, entre 50 et 125°C, pendant 20 à 60 min, de collagène fibreux qui a été préalablement soumis à un traitement réticulant.

17. Peau artificielle selon la revendication 14, dans laquelle ladite couche en contact avec la plaie prend la forme d'une matière spongieuse.

18. Peau artificielle selon la revendication 14, dans laquelle ledit collagène dénaturé contient 0 à 80 % d'hélice.

19. Peau artificielle selon la revendication 14, dans laquelle la fibroïne de ladite couche support prend la forme d'un non-tissé poreux insoluble dans l'eau.

20. Peau artificielle selon la revendication 14, dans laquelle ladite couche contrôlant la perméation de l'eau est en matériaux non toxiques sélectionnés dans le groupe constitué par les résines de silicone, les esters polyacryliques, les esters polyméthacryliques et les polyuréthannes.

21. Peau artificielle selon la revendication 14, dans laquelle ladite couche contrôlant la perméation de l'eau présente un flux hydrique d'environ 0,1 à 1 mg/cm²/h.

22. Peau artificielle selon la revendication 14, dans laquelle au moins une desdites couches : couche en contact avec la plaie, couche support et couche contrôlant la perméation de l'eau, contient un agent antibactérien.

23. Peau artificielle selon la revendication 22, dans laquelle ledit agent antibactérien est sélectionné dans le groupe constitué par la sulfadiazine d'argent, la gentamycine et le nitrate d'argent.

24. Peau artificielle comprenant :
une couche en contact avec la plaie ; et
une couche contrôlant la perméation de l'eau, appliquée sur une face de ladite couche en contact avec la plaie en vue du contrôle de la perméation de l'eau, ladite couche en contact avec la plaie étant en collagène dénaturé obtenu par chauffage, en présence d'eau, entre 50 et 125°C, pendant 20 à 60 min, de collagène qui a été préalablement soumis à un traitement réticulant.

25. Peau artificielle selon la revendication 24, dans laquelle ledit collagène dénaturé est dépourvu de la fraction ayant un pouvoir antigénique à l'extrémité terminale de la molécule de collagène.

26. Peau artificielle selon la revendication 24, dans laquelle ledit collagène dénaturé est préparé par chauffage, en présence d'eau, entre 50 et 125°C, pendant 20 à 60 min, de collagène fibreux qui a été soumis préalablement à un traitement réticulant.

27. Peau artificielle selon la revendication 24, dans laquelle ladite couche en contact avec la plaie prend la forme d'une matière spongieuse.

28. Peau artificielle selon la revendication 24, dans laquelle ledit collagène dénaturé contient 0 à 80 % d'hélice.

29. Peau artificielle selon la revendication 24, dans laquelle ladite couche contrôlant la perméation de l'eau est en un matériau non toxique sélectionné dans le groupe constitué par les résines de silicone, les esters polyacryliques, les esters polyméthacryliques et les polyuréthannes.

30. Peau artificielle selon la revendication 24, dans laquelle ladite couche contrôlant la perméation de l'eau a un flux hydrique compris entre environ 0,1 et 1 mg/cm²/h.

31. Peau artificielle selon la revendication 24, dans laquelle au moins une des deux couches, couche en contact avec la plaie et couche contrôlant la perméation de l'eau, contient un agent antibactérien.

32. Peau artificielle selon la revendication 31, dans laquelle ledit agent antibactérien est sélectionné dans le groupe constitué par la sulfadiazine d'argent, la gentamycine et le nitrate d'argent.
